# EUROPEAN PATENT APPLICATION

(11) **EP 2 682 134 A1**
(43) Date of publication of application: **08.01.2014**
(21) Application number: 12752063.3
(22) Date of filing: 28.02.2012
(51) Int. Cl.: A61L 27/00, C12M 3/04

(54) **CYTOKINE-PRODUCING CELL SHEET AND METHOD FOR USING SAME**

(30) Priority: 28.02.2011 JP 2011043198
(71) Applicant: Tokyo Women's Medical University, Tokyo 162-8666 (JP); Osaka University, Suita-shi, Osaka 565-0871 (JP)
(72) Inventor: KINOOKA, Masahiro, Suita-shi Osaka 565-0871 (JP); SAITO, Atsuhiro, Suita-shi Osaka 565-0871 (JP); SAWA, Yoshiki, Suita-shi Osaka 565-0871 (JP); SHIMIZU, Tatsuya, Tokyo 162-8666 (JP); OKANO, Teruo, Tokyo 162-8666 (JP)
(74) Representative: HOFFMANN EITLE
(86) International application number: PCT/JP2012/054992
(87) International publication number: WO 2012/118099

(57) **Abstract**

The types and relative proportions of cells constituting a cell sheet stack, and the number of cells to be seeded are altered to change the state of the cells in the cell sheet stack, whereby production of an angiogenesis-promoting cytokine as well as construction of a vascular endothelial network can be optimized.

## Description

### TECHNICAL FIELD

The present invention relates to a cell sheet producing a cytokine involved in angiogenesis and a cell sheet having a high-density vascular endothelial cell network constructed therein, both of which are useful in various fields including drug discovery, pharmacy, medicine, and biology, as well as a method for preparing the same and a method for using the same. The present application is an application whose priority is claimed on the basis of the Japanese patent application (application No. 2011-043198) filed on February 28, 2011.

### BACKGROUND ART

In recent years, various regenerative medicine technologies have attracted attention for the regeneration of damaged biological tissues. This can typically be seen from the case of regeneration of myocardial tissues -- in this field, many studies have been made concerning, for example, a method for regenerating a myocardial tissue by directly injecting cardiomyocytes, skeletal myoblasts, or mesenchymal stem cells thereinto, and a below-mentioned regenerative therapy characterized by transplanting a myocardial tissue cell sheet with low damage which is obtained by culturing cardiomycocytes on a special temperature-responsive substrate and making a change to temperature (refer to Patent Document 1); further, clinical studies in humans have been started on some types of cells.

Patent Document 2 discloses a method for using, as a graft, cells selected from cardiomycocytes, fibroblasts, smooth muscle cells, endothelial cells and skeletal myoblasts for the purpose of improving a myocardial function, promoting angiogenesis, and regenerating a myocardial tissue. Patent Document 3 discloses a method for using a skeletal myocyte composition as a graft for the purpose of restoring a cardiac function. According to Patent Documents 2 and 3, animal studies showed that cardiac functions were restored by injecting the above-mentioned grafts, thereby demonstrating the effectiveness of these approaches. However, the cell grafts disclosed in Patent Documents 2 and 3 are both injected in the form of a cell suspension, and the transplanted cells have some problems as mentioned below: it is difficult to control a transplantation site because some transplanted cells may flow out of the transplantation site; and no transplantation can be performed effectively because some transplanted cells may undergo necrosis. Thus, further improvement has been desired.

One of the solutions developed for the above-mentioned problems is a transplantation method using a cell sheet. Cells that are to serve as a graft are cultured in the form of sheet beforehand and then the cells are transplanted onto a biological tissue with depressed function, whereby outflow of the cells into areas around a transplantation site, which has been encountered by conventional cell suspension injection methods, can be minimized; thus, this method has attracted attention as a new technology in regenerative medicine.

Many types of cell sheets for use in transplantation are prepared using particularly anchorage-dependent cells among animal cells including human cells. In order to prepare a cell sheet, animal cells must be first cultured *in vitro* and temporarily adhered on the surface of a substrate, and the cultured cells must be detached from the substrate while not disintegrating but maintaining the form which they took during culture on the surface of the substrate.

As regards cell sheet preparation methods, Patent Document 1 discloses a novel cell culture method in which cells are placed on a cell culture support having a substrate surface coated with a polymer whose upper or lower critical solution temperature in water is 0 to 80°C, the cells are cultured at a temperature either below the upper critical solution temperature or above the lower critical solution temperature, and then the temperature is brought to a temperature either above the upper critical solution temperature or below the lower critical solution temperature, so that the cultured cells are detached without enzymatic treatment. Patent Document 4 discloses that dermal cells are cultured using the above-mentioned temperature-responsive cell culture substrate at a temperature either below the upper critical solution temperature or above the lower critical solution temperature, and then the temperature is brought to a temperature either above the upper critical solution temperature or below the lower critical solution temperature, so that the cultured dermal cells are detached with little damage. The use of such temperature-responsive cell culture substrates has allowed various innovations of conventional culture technologies.

Further, Patent Document 5 reports the following findings: myocardial tissue cells are cultured on a cell culture support having a substrate surface coated with a temperature-responsive polymer to produce a myocardium-like cell sheet, the temperature of a medium is brought to a temperature either above the upper critical solution temperature or below the lower critical solution temperature, the cultured cell sheet stack is brought into close contact with a polymer membrane, and the cell sheet is detached together with the polymer membrane, whereby a cell sheet can be constructed which has few structural defects and which is furnished with several capabilities of an *in vitro* myocardium-like tissue; and the resulting sheet is structured three-dimensionally using a specified method to thereby construct a three dimensional structure composed of such sheets. However, these methods have a problem in that stratifying of an infinite number of cell sheets is not possible -- in the case of myocardium-like cell sheets, they can only be piled up in about 3 layers at the maximum or to a thickness of about 100 µm at the maximum, and those cells which are piled up to a greater thickness will not be viable if they only rely on the diffusion of a medium or an interstitial fluid. In order to prepare a cell sheet having a greater thickness, it is essential to develop a technique for preparing a cell sheet having a vascular network that enables delivery of oxygen and nutrients throughout a three-dimensionally structured tissue; and developing such a technique has been a common problem to researchers in regenerative medicine.

The efficacy of a cell sheet on the myocardium was confirmed by the fact that a rat cardiomyocyte sheet transplanted onto a rat model of myocardial infarct improved a cardiac function depressed by ischemia (Non-patent Document 1). The efficacies of other cell sheets derived from various cell types were also demonstrated -- for example, it was confirmed that myoblast sheets acting as a clinically applicable cell type as well as mesenchymal stem cell sheets derived from an adipose tissue or a menstrual blood improved a cardiac function (Non-patent Documents 2, 3 and 4).

It was suggested that improvement in a cardiac function may be achieved due to the following mechanism: a myoblast-derived cell sheet transplanted onto a diseased site stably and continuously secretes various cytokines including vascular endothelial growth factor (VEGF) and hepatocyte growth factor (HGF), which promote angiogenesis, as well as secretes stromal cell-derived factor 1 (SDF-1) and the like, which recruit stem cells to areas around a transplantation site of a cell sheet (paracrine effect). It is believed that the cytokines secreted from the cell sheet act on a stacked cell sheet per se, affecting the construction of a vascular network inside the stacked cell sheet (autocrine effect).

Under the circumstances, Non-patent Document 5 reports a method for preparing a layered cell sheet having a thickness of 100 µm or greater -- it reported that cell sheets are stacked *in vivo* and, after neovascularization takes place in the cell sheets, further cell sheet laminating is performed, and then these steps are repeated, whereby a layered cardiomyocyte sheet having a thickness of 1 mm can be prepared. It suggests that in order to produce a thick cell sheet, it is essential that nutrients and oxygen be supplied to individual cells in the cell sheets. In order to prepare a layered cell sheet provided with a vascular network by the method disclosed in Non-patent Document 5, cell sheet laminating must be performed *in vivo* repeatedly, so vascular network formation must be induced from the side of a recipient's body. It is also necessary to incise a transplantation site every time cell sheet laminating is performed; accordingly, this method imposes a significant burden on recipients.

In order to prepare a cell sheet stack having a thickness greater than 100 µm *in vitro,* it is essential to develop a technique for providing the sheet with a vascular network *in vitro.* It was shown that co-culture of rat cardiomyocytes and vascular endothelial cells produces a cell sheet stack composed of cell sheets having a vascular endothelial cell network structure formed therein. And, when the resulting cell sheet stack is transplanted into a recipient's body, capillary vessels originating from the vascular endothelial cell network in the cell sheets are induced in areas around a transplantation site, thereby enabling regeneration of a thicker myocardial tissue (Non-patent Document 6). Construction of a vascular endothelial network enables not only preparation of a thick cell sheet stack but also effective secretion of various cytokines via the formed vascular network to tissues around a transplantation site; so, significant enhancement of the therapeutic effect after transplantation can also be expected.

As mentioned above, in order to achieve preparation *in vitro* of a tissue mimicking a biological tissue in this technical field, a technique for providing the tissue with a vascular network is essential, so further improvement is needed. However, conventional attempts to construct a three-dimensional biological tissue as exemplified by a stratified cell sheet have lacked a method by which a vascular endothelial cell network constructed in a three-dimensional biological tissue can be evaluated in a quantitative and simple manner. Hence, no report has been made of a method for designing a stratified cell sheet having an optimum vascular endothelial network constructed therein. Thus, the present inventors developed a technique for evaluating a vascular endothelial cell network constructed as a three-dimensional structure in a stratified cell sheet by using a two-dimensional analytical method (Patent Document 6). This technique made it possible to simply evaluate a vascular endothelial cell network in a cell sheet stack. It is believed that successful contrivance of a method for designing a cell sheet stack having a high-density vascular endothelial network that has been constructed therein beforehand will lead to possibilities not only to stably prepare a thick cell sheet stack *in vitro* but also to produce a cell sheet stack that sufficiently promotes angiogenesis to potentially exhibit a satisfactory therapeutic effect.

### CITATION LIST

### PATENT DOCUMENT

[Patent Document 1] Japanese Unexamined Patent Application Publication No. H02-211865
[Patent Document 2] Japanese Patent Domestic Publication No. 2002-518006
[Patent Document 3] Japanese Patent Domestic Publication No. 2003-505475
[Patent Document 4] Japanese Unexamined Patent Application Publication No. H05-192138
[Patent Document 5] Japanese Patent Domestic Re-publication No. 2002-008387
[Patent Document 6] International Patent Publication No. 2010/101225

### NON-PATENT DOCUMENT

[Non-patent Document 1] Transplantation, 80, 1586-1595, 2005
[Non-patent Document 2] J. Thorac. Cardiovasc. Surg., 130, 1333-1341 (2005)
[Non-patent Document 3] Nat. Med., 12, 459-465 (2006)
[Non-patent Document 4] Stem Cells, 26, 1695-1704 (2008)
[Non-patent Document 5] FASEB. J., 20 (6), 708-710 (2006)
[Non-patent Document 6] J. Biochim. Biophysic. Res. Commun., 341, 573-582 (2006)

### SUMMARY OF INVENTION

### TECHNICAL PROBLEM

The present invention has been made for the purpose of establishing a method for preparing a cell sheet stack having an ability to produce an angiogenesis-promoting cytokine and/or having a high-density vascular endothelial network constructed therein. In other words, this invention is such that makes it possible to provide a cell sheet stack that is superior in the ability to produce an angiogenesis-promoting cytokine and/or has a higher-density vascular endothelial network constructed therein, as compared to conventional methods.

### SOLUTION TO PROBLEM

In order to solve the above-mentioned problems, the present inventors has made research and development with investigations being conducted from different angles. As a result, the inventors found that a cell sheet stack prepared by using a temperature-responsive culture substrate generates a special environment resembling a structure in the living body. Furthermore, the inventors found that the types and relative proportions of cells constituting the cell sheet stack, and the number of cells to be seeded are altered to change the state of the cells in the cell sheet stack, whereby production of an angiogenesis-promoting cytokine as well as construction of a vascular endothelial network can be optimized. The present invention has been completed on the basis of these findings.

More specifically, the present invention provides: a cell sheet having an ability to produce a cytokine involved in angiogenesis promotion, wherein cells constituting the cell sheet include at least fibroblasts as well as include skeletal myoblasts in a proportion of 10% or higher, and/or wherein the cell sheet has a vascular endothelial cell network constructed therein; or a stack thereof; and a method for using the same.

### ADVANTAGEOUS EFFECTS OF INVENTION

The inventive method for preparing a cell sheet stack having an ability to produce a cytokine involved in angiogenesis promotion or a cell sheet stack having an ability to produce a cytokine involved in angiogenesis promotion and having a vascular endothelial cell network constructed therein makes it possible to prepare a cell sheet or cell sheet stack each having a higher ability to produce a cytokine involved in angiogenesis and/or having a higher-density vascular endothelial network constructed therein even under *in vitro* environment, as compared to conventionally available cell sheets or cell sheets. Also, the cell sheet or cell sheet stack each prepared by using the present invention can serve as a graft having a high ability to restore a cardiac function.

### BRIEF DESCRIPTION OF DRAWINGS

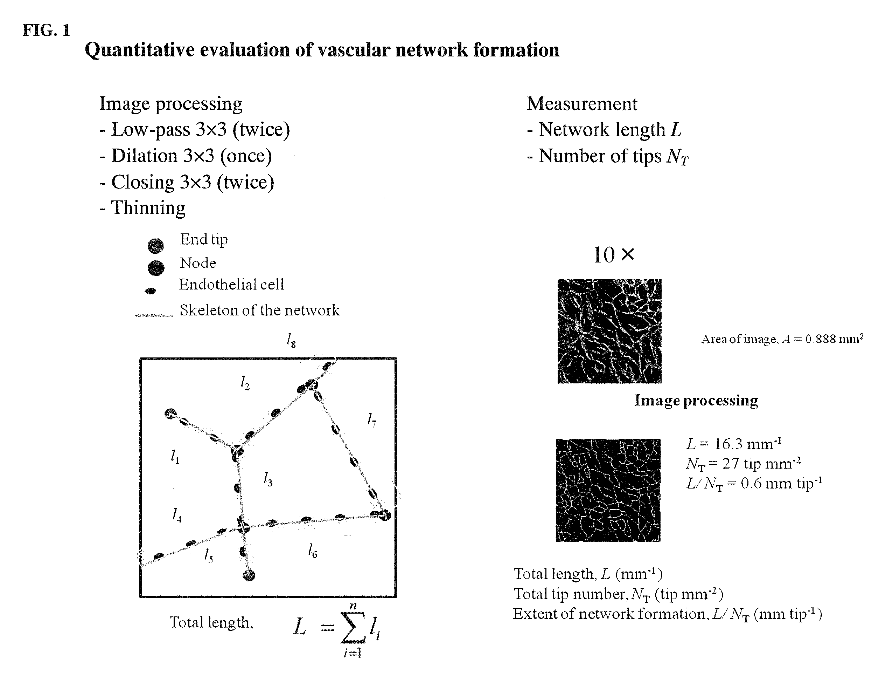

[FIG. 1] FIG. 1 shows the method for the quantitative evaluation of vascular network formation performed in Example 1 or 3. The areas shown in green (if displayed in black and white, the meshed areas shown in light gray) are vascular endothelial cells.

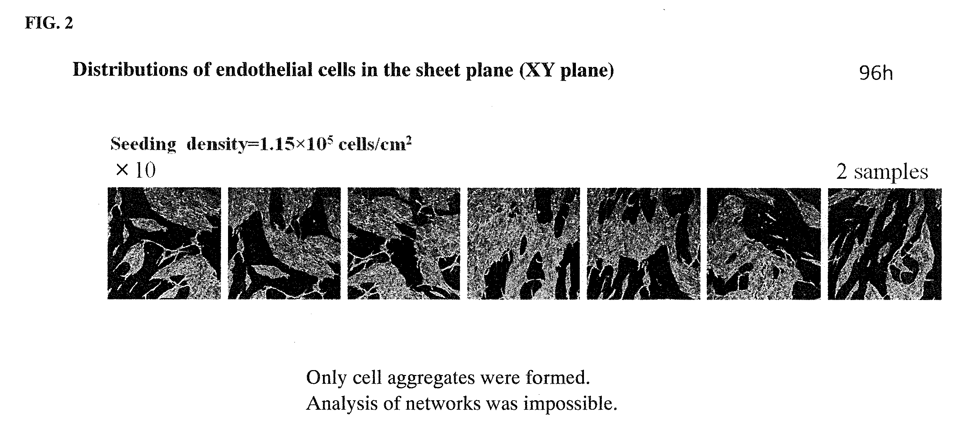

[FIG. 2] FIG. 2 shows how a myoblast density influenced the formation of a vascular endothelial cell network in a cell sheet stack in Example 1. The areas shown in green (if displayed in black and white, the meshed areas shown in light gray) are vascular endothelial cells. The results shown are for the case of the seeding density of 1.15×10⁵ cells/cm².

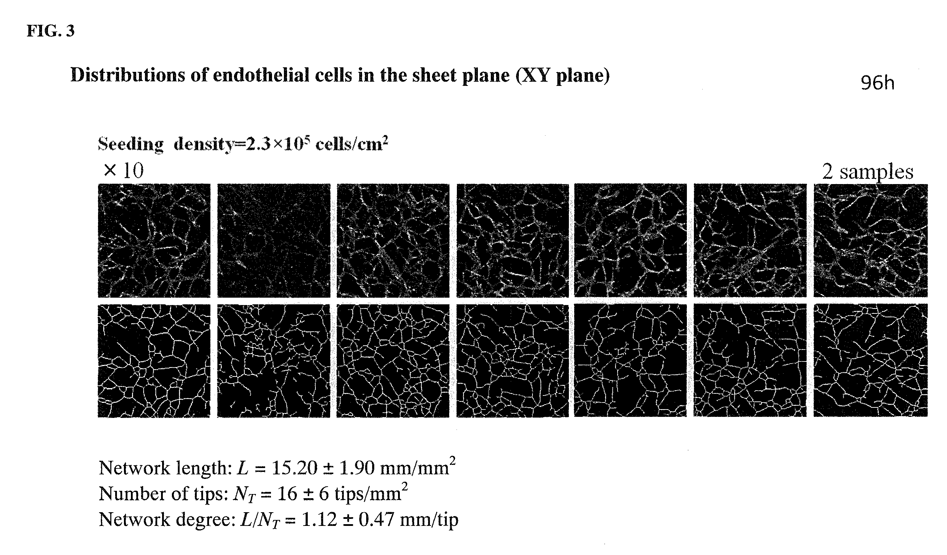

[FIG. 3] FIG. 3 shows how a myoblast density influenced the formation of a vascular endothelial cell network in a cell sheet stack in Example 1. The areas shown in green (if displayed in black and white, the meshed areas shown in light gray) are vascular endothelial cells. The results shown are for the case of the seeding density of 2.3×10⁵ cells/cm².

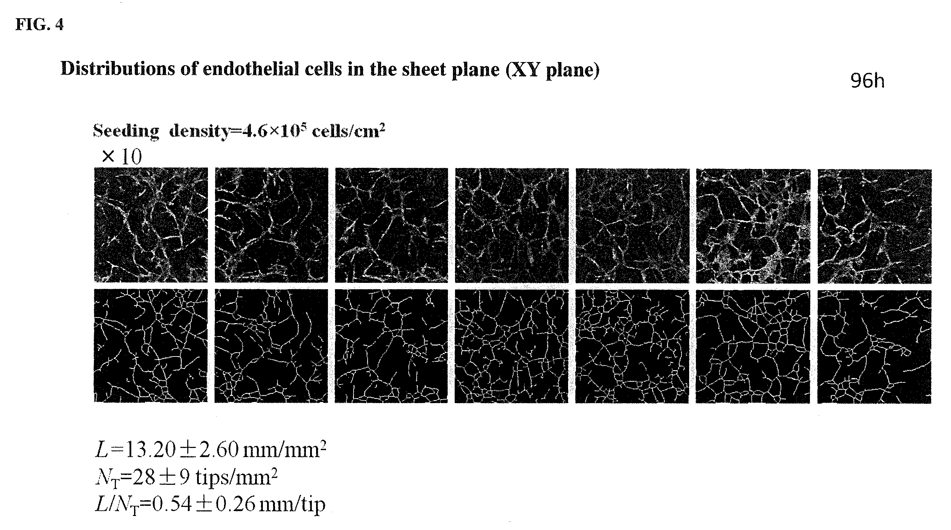

[FIG. 4] FIG. 4 shows how a myoblast density influenced the formation of a vascular endothelial cell network in a cell sheet stack in Example 1. The areas shown in green (if displayed in black and white, the meshed areas shown in light gray) are vascular endothelial cells. The results shown are for the case of the seeding density of 4.6×10⁵ cells/cm².

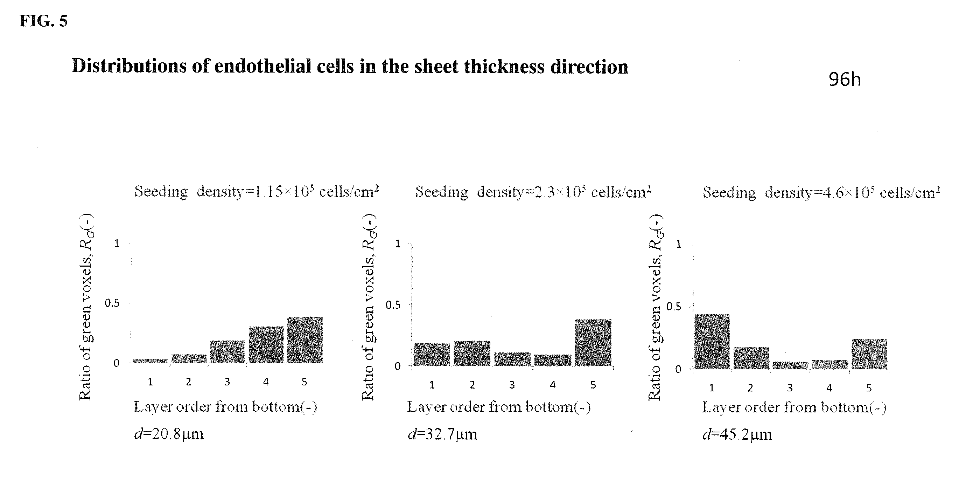

[FIG. 5] FIG. 5 shows how myoblast densities influenced the formation of a vascular endothelial cell network in cell sheets in Example 1. The graphs show the proportions of the vascular endothelial cells present in individual cell sheet layers in the Z-axis direction. The numbers, 1-5, shown below the respective graphs represent the layer numbers assigned, with the bottommost cell sheet being indicated as layer 1. The symbol "d" represents the thickness of a layered cell sheet.

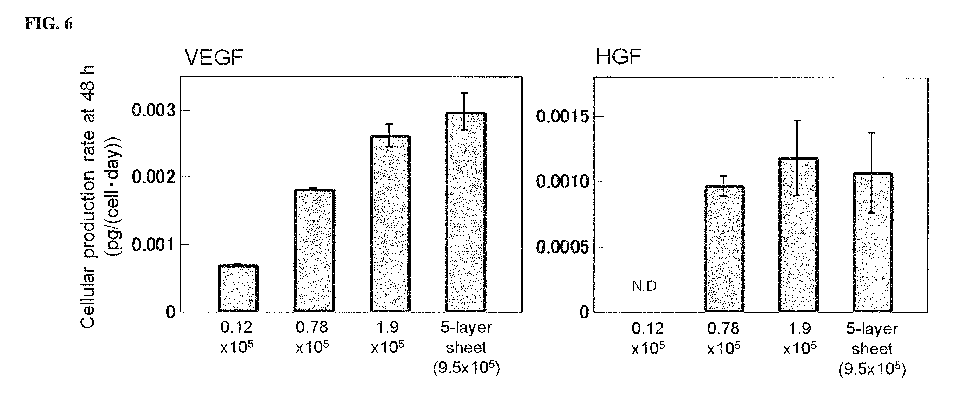

[FIG. 6] FIG. 6 shows how myoblast densities influenced the abilities to produce angiogenesis-promoting cytokines in Example 2. The graphs show the cytokine production levels (pg) per cell · day at 48 hours of culture.

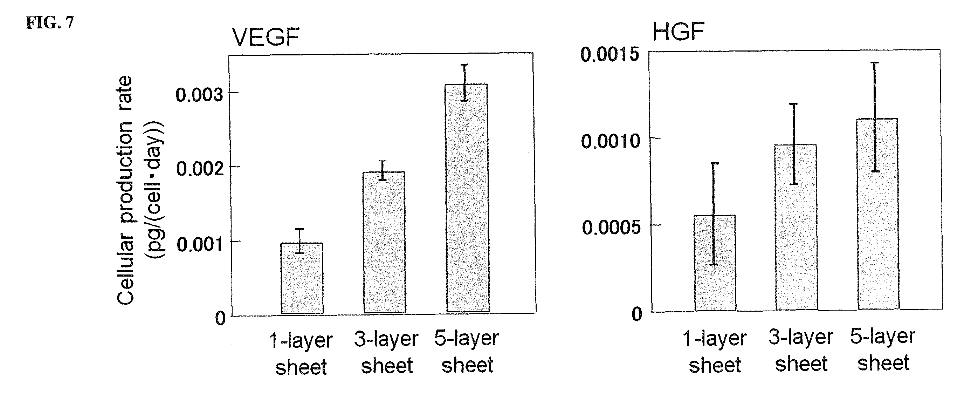

[FIG. 7] FIG. 7 shows how numbers of stacked monolayer myoblast sheets influenced the abilities to produce angiogenesis-promoting cytokines in Example 2. The graphs show the cytokine production levels (pg) per cell · day at 48 hours of culture.

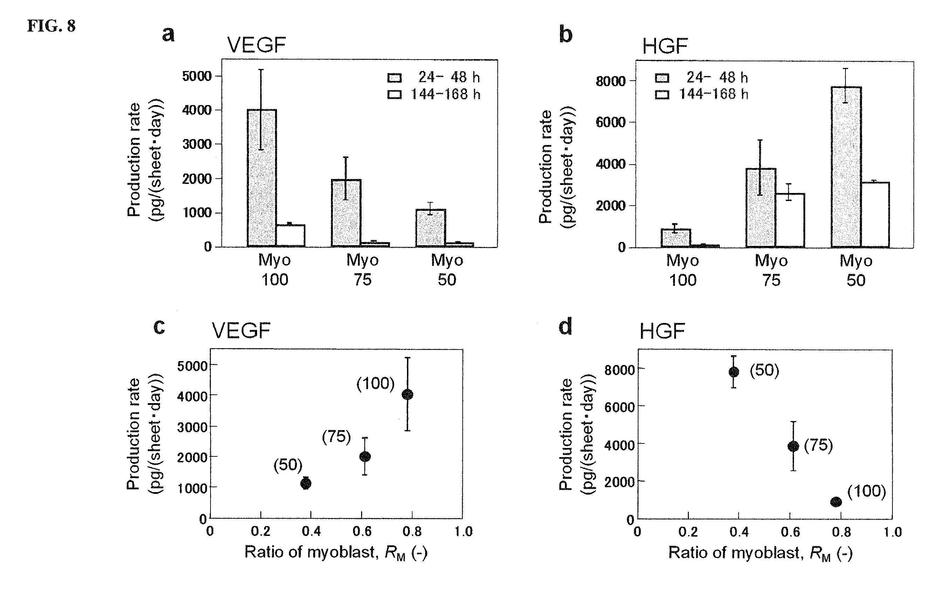

[FIG. 8] FIG. 8 shows the levels of production of angiogenesis-promoting cytokines (VEGF, HGF) per cell · day for the different periods (from 24 hours to 48 hours, and from 144 hours to 168 hours) of culture of a mixture of myoblasts and fibroblasts in Example 2 (FIGs. 8a, 8b). The numbers shown below the abbreviation "Myo" (which means myoblasts) represent the proportions of the myoblasts incorporated in the process of cell seeding. FIGs. 8c and 8d show the results of correcting the results of FIGs. 8a and 8b using the actual myoblast proportion.

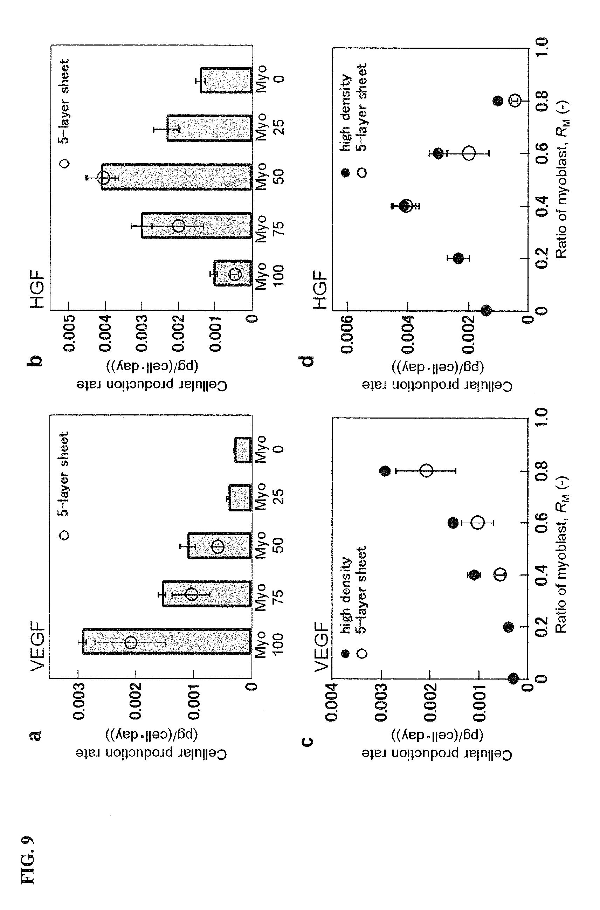

[FIG. 9] FIG. 9 shows the levels of production of cytokines (VEGF, HGF) per cell/day in cell sheets and cell sheet stacks composed of a mixture of myoblasts ("Myo") and fibroblasts in Example 2 (FIGs. 9a, 9b). The numbers shown below the abbreviation "Myo" (which means myoblasts) represent the proportions of the myoblasts incorporated in the process of cell seeding. The red circles shown in FIGs. 9a and 9b (if displayed in black and white, the circles shown in the bars of the graphs) show the cytokine production levels per cell/day in 5-layered cell sheets. FIGs. 9c and 9d show the results of correcting the results of FIGs. 9a and 9b using the actual myoblast proportion.

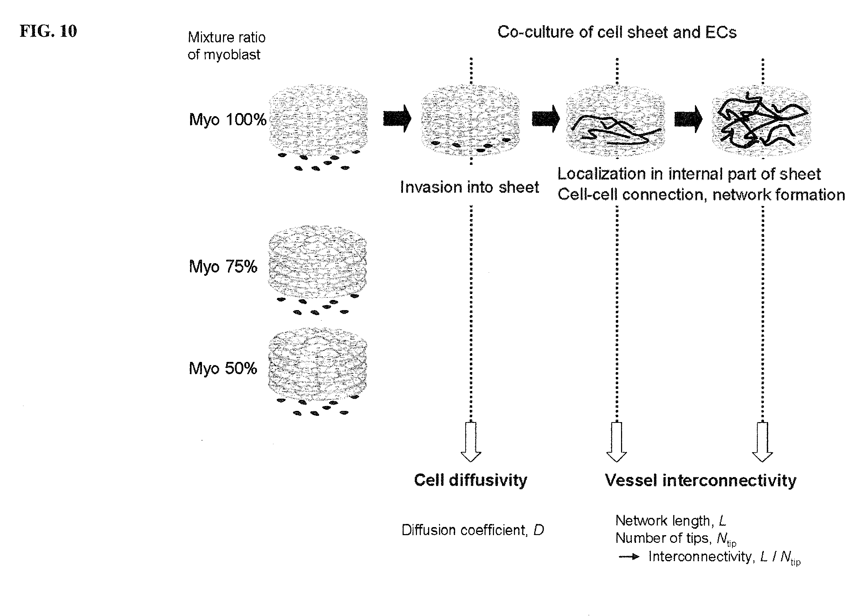

[FIG. 10] FIG. 10 is a conceptual diagram showing how relative proportions of myoblasts and fibroblasts influenced the formation of a vascular endothelial cell network in cell sheet stacks in Example 3.

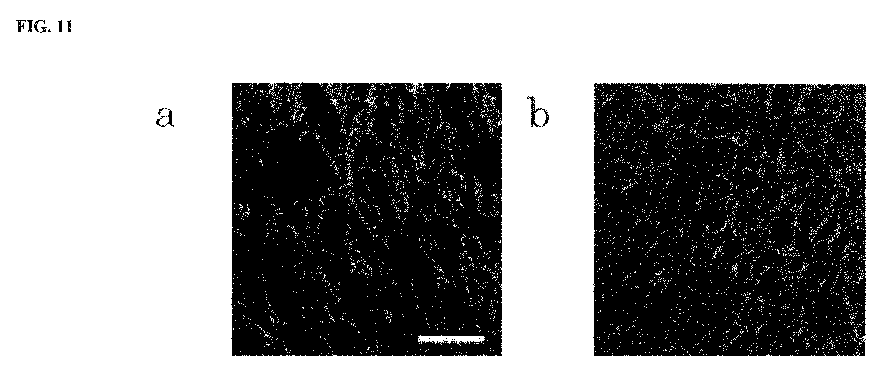

[FIG. 11] FIG. 11 is a pair of photographs showing how relative proportions of myoblasts and fibroblasts influenced the formation of a vascular endothelial cell network in cell sheets in Example 3. FIG. 11a is a photograph showing the vascular endothelial network constructed in the cell sheet composed exclusively of myoblasts (the green areas (if displayed in black and white, the meshed areas shown in light gray)); and FIG. 11b is a photograph showing the vascular endothelial cell network constructed in the cell sheet composed of 50% myoblasts and 50% fibroblasts (the green areas (if displayed in black and white, the meshed areas shown in light gray)).

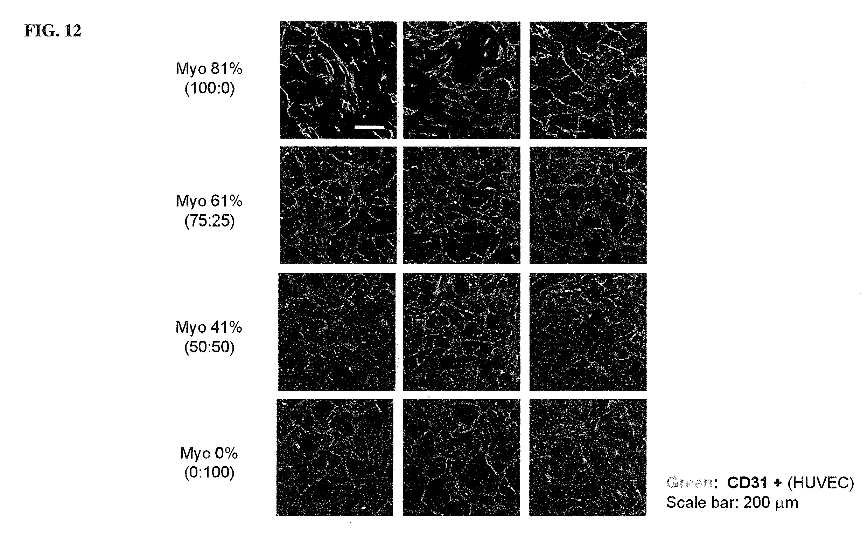

[FIG. 12] FIG. 12 is a set of photographs showing how relative proportions of myoblasts and fibroblasts influenced the formation of a vascular endothelial cell network in 5-layered cell sheets in Example 3. FIG. 12a is a photograph showing the vascular endothelial cell network constructed in a cell sheet composed of 81% myoblasts and 19% fibroblasts (the green areas (if displayed in black and white, the meshed areas shown in light gray)); FIG. 12b is a photograph showing the vascular endothelial cell network constructed in a cell sheet composed of 61% myoblasts and 39% fibroblasts (the green areas (if displayed in black and white, the meshed areas shown in light gray)); FIG. 12c is a photograph showing the vascular endothelial cell network constructed in a cell sheet composed of 41% myoblasts and 59% fibroblasts (the green areas (if displayed in black and white, the meshed areas shown in light gray)); and FIG. 12d is a photograph showing the vascular endothelial cell network constructed in a cell sheet composed of 100% fibroblasts (the green areas (if displayed in black and white, the meshed areas shown in light gray)).

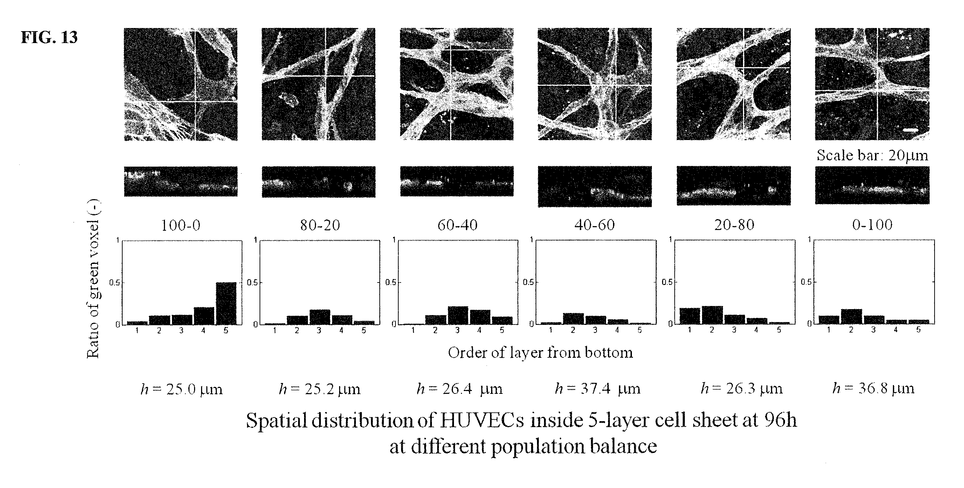

[FIG. 13] FIG. 13 is a set of photographs showing how relative proportions of myoblasts and fibroblasts influenced the formation of a vascular endothelial cell network in 5-layered cell sheets in Example 3. The figures in the first row show the appearances of the vascular endothelial networks (the green areas (if displayed in black and white, the meshed areas shown in light gray)) in the XY-axis direction. The figures in the second row show the appearances of the vascular endothelial networks (the green areas (if displayed in black and white, the meshed areas shown in light gray)) in the Z-axis direction. The graphs in the third row show the proportions of the vascular endothelial cells present in individual cell sheet layers. All of the figures show the appearances of the vascular endothelial networks at 96 hours of culture after completion of cell sheet stratifying. The cell sheet which is the further to the left had more myoblasts seeded in the process of cell sheet preparation, and the layered cell sheet which is the further to the right had more fibroblasts (the proportion of myoblasts is 100% on the leftmost column and decreases to 80%, 60%, 40%, 20%, and 0%). The numbers, 1-5, shown below the respective graphs represent the layer numbers assigned, with the bottommost cell sheet being indicated as layer 1. The symbol "h" represents the thickness of a layered cell sheet.

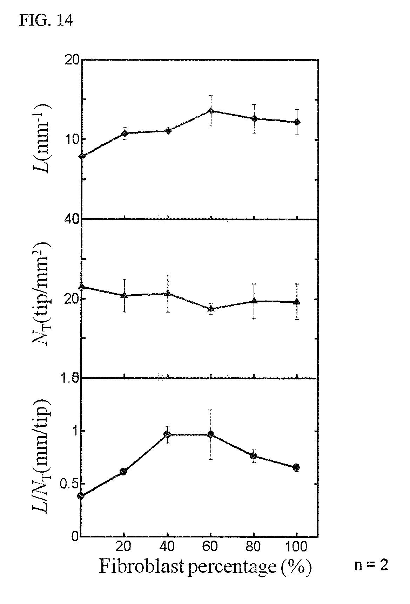

[FIG. 14] FIG. 14 shows the degrees of construction of vascular endothelial cell networks in the layered cell sheets composed of a mixture of myoblasts and fibroblasts in Example 3. The symbol "L" represents the total network length per 1 mm²; the symbol "N_{T}" represents the number of tips in the network per 1 mm²; and the symbol "L/N_{T}" represents the value of L divided by N_{T}. The shaded area shows the relative proportion range in which an optimum vascular endothelial network is formed in layered cell sheets composed of myoblasts and fibroblasts.

### DESCRIPTION OF EMBODIMENTS

The present invention relates to a cell sheet or cell sheet stack each secreting a cytokine involved in angiogenesis promotion and/or having a high-density vascular endothelial cell network constructed therein, as well as a method for using the same. The cell sheet stack provided by this invention produces at least one angiogenesis-promoting cytokine selected from vascular endothelial growth factor (VEGF), hepatocyte growth factor (HGF), fibroblast growth factor (FGF), and epidermal growth factor (EGF) at higher concentrations than cell sheets prepared by conventional methods. This invention can provide a cell sheet stack secreting a cytokine involved in angiogenesis promotion at high concentrations and/or having an optimized vascular endothelial cell network.

The technique preferably used for evaluating the high-density vascular endothelial cell network constructed in the cell sheet stack prepared by the present invention is a cell evaluation system through two-dimensional analysis of a cell sheet stack and target cells, in which a biological parameter relating to at least one selected from viability, proliferation, migration, and differentiation of the target cells is obtained by a two-dimensional analytical technique (refer to the following patent document: International Patent Publication No. 2010/101225). The two-dimensional analyzer is not particularly limited as long as it is a technique for obtaining two-dimensional or planar information of cells, and examples of the analyzer include microscopes such as fluorescence microscope, optical microscope, stereoscopic microscope, laser microscope, and confocal microscope; plate readers; and other devices having macro lenses. Observation may be made on images or visually under a microscope. The analytical technique is not particularly limited as long as it is an ordinary method using such an analyzer as mentioned above, and examples include methods in which fluorescence staining and/or dye staining of cells are/is performed by at least one means selected from reagents, proteins, genes, and the like, and the degree of staining is observed typically using such a microscope as mentioned above. Labeled cells means the cells subjected to said fluorescence staining and/or dye staining. It is desirable to evaluate the cell sheet stack prepared by this invention using such an evaluation method as mentioned above, because the evaluation makes it possible to confirm that a cell sheet or cell sheet stack each characterized by producing an angiogenesis-promoting cytokine at a high level and/or by having an optimum vascular endothelial network constructed therein has been successfully prepared in a consistent manner.

In order to prepare the cell sheet stack according to the present invention, a temperature-responsive substrate is required. This substrate is a cell culture substrate that has a specific surface and which is grafted with a temperature-responsive polymer by electron beam irradiation. The material to be used for the substrate is not particularly limited, but the substrate serving as a cell adhesion surface is made of polystyrene, polycarbonate, polymethyl methacrylate, or a combination of two or more of them. Particularly preferred is polystyrene, which is usually used for cell culture substrates. The temperature-responsive polymer that is used for coating the substrate has an upper or lower critical solution temperature of 0 to 80°C, more preferably 20 to 50°C, in an aqueous solution. An upper or lower critical solution temperature higher than 80°C is undesirable since it may cause death of cells. An upper or lower critical solution temperature lower than 0°C is also undesirable since it usually causes an extreme decrease in cell growth rate or death of cells.

The temperature-responsive polymer used in the present invention may be a homopolymer or a copolymer. Examples of these polymers include polymers described in Japanese Unexamined Patent Application Publication No. H02-211865. Specifically, such polymers are typically prepared by homopolymerization or copolymerization of the following monomers. Examples of monomers that can be used include (meth)acrylamide compounds, N-(or N,N-di)alkyl-substituted (meth)acrylamide derivatives, and vinyl ether derivatives. Copolymers can be made of any two or more of the above-mentioned monomers. Alternatively, they may be prepared by copolymerization of any of the above-mentioned monomers with any other monomer than said monomers, or by graft polymerization or copolymerization of polymers. A mixture of polymers or copolymers may also be used. Such polymers may also be crosslinked as long as their inherent properties are not impaired. Considering that separation takes place at a temperature ranging from 5 to 50°C since it is cells that are to be cultured and detached on the temperature-responsive polymer, the polymer can be exemplified by poly-N-n-propylacrylamide (homopolymer's lower critical solution temperature, 21°C), poly-N-n-propylmethacrylamide (ditto, 27°C), poly-N-isopropylacrylamide (ditto, 32°C), poly-N-isopropylmethacrylamide (ditto, 43°C), poly-N-cyclopropylacrylamide (ditto, 45°C), poly-N-ethoxyethylacrylamide (ditto, ≈35°C), poly-N-ethoxyethylmethacrylamide (ditto, ≈45°C), poly-N-tetrahydrofurfurylacrylamide (ditto, ≈28°C), poly-N-tetrahydrofurfurylmethacrylamide (ditto, ≈35°C), poly-N,N-ethylmethylacrylamide (ditto, 56°C), and poly-N,N-diethylacrylamide (ditto, 32°C). Exemplary monomers for copolymerization that are used in this invention include, but are not particularly limited to, polyacrylamide, poly-N,N-diethylacrylamide, poly-N,N-dimethylacrylamide, polyethylene oxide, polyacrylic acid and a salt thereof, and hydrated polymers such as polyhydroxyethyl methacrylate, polyhydroxyethyl acrylate, polyvinyl alcohol, polyvinyl pyrrolidone, cellulose, and carboxymethyl cellulose.

The method used for coating the surface of the substrate with such a polymer as mentioned above in the present invention is not particularly limited, and the coating can typically be performed by subjecting the substrate and said monomer or polymer to electron beam (EB) irradiation, γ-ray irradiation, ultraviolet ray irradiation, plasma treatment, corona treatment, or organic polymerization reaction, or by physical adsorption through spread coating, kneading or the like. The coating amount of the temperature-responsive polymer on the surface of the culture substrate can be in the range of 1.1 to 2.3 *µ*g/cm², preferably 1.4 to 1.9 *µ*g/cm² and more preferably 1.5 to 1.8 *µ*g/cm². A coating amount lower than 1.1 *µ*g/cm² is undesirable since it makes it difficult for cells to be detached from the polymer even if a stimulus is applied, leading to a significant deterioration in work efficiency. Also, a coating amount higher than 2.3 *µ*g/cm² makes it difficult for cells to adhere to the area of interest, preventing them from adhering adequately. In such as case, if cell adhesive proteins are further applied onto the temperature-responsive polymer coating layer, the coating amount of the temperature-responsive polymer on the substrate surface can be higher than 2.3 *µ*g/cm², but this coating amount is advantageously not higher than 9.0 *µ*g/cm², preferably not higher than 8.0 *µ*g/cm², and more preferably not higher than 7.0 *µ*g/cm². A coating amount higher than 9.0 *µ*g/cm² is undesirable since it makes it difficult for cells to adhere even if such cell adhesive proteins are further applied onto the temperature-responsive polymer coating layer. The cell adhesive proteins can be of any type without particular limitation, and may be any one of collagen, laminin, laminin 5, fibronectin, matrigel and the like, or a mixture of two or more of them. The cell adhesive proteins can be applied according to any conventional method, and are commonly applied by coating the substrate surface with the cell adhesive proteins in aqueous solution, removing the aqueous solution, and performing rinsing. This invention is a technique using a temperature-responsive culture dish with the aim of putting a cell sheet itself to use to the extent possible. Therefore, an extremely large coating amount of the cell adhesive proteins on the temperature-responsive polymer layer is undesirable. The coating amounts of the temperature-responsive polymer and the cell adhesive proteins can be measured according to any conventional method; for example, they may be measured by directly measuring the area to which cells adhere using the FT-IR-ATR method, or by a method in which a polymer that has been labeled beforehand is immobilized using the same procedure and the amount of the labeled polymer immobilized on the area to which cells adhere is determined to estimate the coating amounts, and any other methods may also be used.

In the present invention, a sheet of cultured cells can be detached and harvested from the temperature-responsive substrate by bringing the temperature of the culture substrate to which the cultured cells adhere to a temperature either above the upper critical solution temperature or below the lower critical solution temperature of the coating polymer on the culture substrate. The detachment may be performed in a medium or any other isotonic solution -- the solution to be used can be selected depending on the purpose. For the purpose of detaching and harvesting the cells more quickly and efficiently; any various methods including lightly tapping or shaking the substrate, or stirring a culture medium with a pipette may be used alone or in combination.

The above-mentioned detachment and harvesting method is now described by referring to the case of using poly(N-isopropylacrylamide) as the temperature-responsive polymer. Poly(N-isopropylacrylamide) is known as a polymer having a lower critical solution temperature of 31°C. When in a free state, it dehydrates at a temperature higher than 31°C in water, and the polymer chains aggregate to cause cloudiness. In contrast, at a temperature of 31°C or lower, the polymer chains hydrate to become dissolved in water. In the present invention, a coating of such a polymer is applied and immobilized onto the surface of a substrate such as a culture dish. Accordingly, at a temperature higher than 31°C, the polymer on the substrate surface dehydrates as mentioned above, but since the coating of the polymer chains has been applied and immobilized on the substrate surface, the latter becomes hydrophobic. On the other hand, at a temperature of 31°C or lower, the polymer on the substrate surface hydrates, whereupon the substrate surface becomes hydrophilic because the polymer chains has been applied and immobilized onto the substrate surface. The hydrophobic surface is an adequate surface for cells to adhere and grow, whereas the hydrophilic surface is such a surface that prevents cells from adhering; thus, cultured cells or cell sheets can be detached simply by cooling.

The substrate to be coated can be any ordinary substrate that can be given a shape, including compounds that are commonly used in cell culture, such as glass, modified glass, polystyrene, and polymethylmethacrylate. Any other polymeric compounds than the above-listed ones as well as all kinds of ceramics can also be used.

The form of the culture substrate in the present invention is not particularly limited, and examples include forms like a dish, a multiplate, a flask, a cell insert used for culture on a porous membrane, and a flat membrane. The substrate to be coated can be any ordinary substrate that can be shaped, including compounds that are commonly used in cell culture, such as glass, modified glass, polystyrene, and polymethylmethacrylate. Any other polymeric compounds than the above-listed ones as well as all kinds of ceramics can also be used.

Also, the method for coating the culture substrate with the temperature-responsive polymer is not particularly limited, and the coating can typically be performed according to the method disclosed in Japanese Unexamined Patent Application Publication No. H02-211865. More specifically, the coating can be performed by subjecting the substrate and such a monomer or polymer as mentioned above to electron beam (EB) irradiation, γ-ray irradiation, ultraviolet ray irradiation, plasma treatment, corona treatment, or organic polymerization reaction, or by physical adsorption through spread coating, kneading or the like.

The cell sheet stack provided in the present invention can be a stack composed either of cell sheets of a single cell type or a combination of cell sheets of different cell types. It is preferable to use two or more different types of cells because interaction between different types of cells yields a cell sheet with varied cell mobility or a cell sheet highly producing an angiogenesis-promoting cytokine. The cells to be used in this invention are not particularly limited, but a cell sheet or cell sheet stack each composed of a combination of high-mobility and low-mobility cells is desirable, because the mobility in the cell sheet or cell sheet stack affects the degree of maturity in construction of a vascular endothelial cell network. Exemplary high-mobility cells include, but are not particularly limited to, skeletal myoblasts, vascular endothelial cells, mesenchymal stem cells, cardiomyocytes, and epidermal basement membrane cells. Exemplary low-mobility cells include, but are not particularly limited to, fibroblasts and differentiated epidermal keratinocytes. Combining of high-mobility and low-mobility cells enables preparation of cell sheet stacks having different mobilities. The combination of high-mobility and low-mobility cells can be, for example, a combination of muscular tissue cells and fibroblasts -- to be specific, a combination of skeletal myoblasts and fibroblasts or a combination of cardiomyocytes and fibroblast. In the process of preparing a cell sheet composed of the combination of muscular tissue cells and fibroblasts, these cells should be mixed in the following proportions: 90-25% muscular tissue cells with the balance being fibroblasts, preferably 75-25% muscular tissue cells with the balance being fibroblasts, most preferably 60-40% muscular tissue cells with the balance being fibroblasts. The cell sheet prepared by mixing different types of cells in the optimum proportions according to this invention is preferred, since this cell sheet produces at least one angiogenesis-promoting cytokine selected from vascular endothelial growth factor (VEGF), hepatocyte growth factor (HGF), fibroblast growth factor (FGF), and epidermal growth factor (EGF) at high concentrations, and has the optimum mobility for the place where a vascular endothelial cell network is constructed.

The term "VEGF" represents vascular endothelial growth factor and generally refers to a glycoprotein that binds as a ligand to a vascular endothelial growth factor receptor (VEGFR) found on the surface of a vascular endothelial cell. VEGF is known as a factor that stimulates proliferation, migration, and differentiation, promotes the activities of proteases such as plasminogen activators and collagenases, and all steps of formation of a vessel-like structure, so-called angiogenesis, in a collagen gel, and also promotes angiogenesis *in vivo.* VEGF not only has a function of exacerbating vascular permeability of microvessels but also is involved in activation of monocytes and macrophages. In an embodiment of this invention, VEGF is mainly secreted from skeletal myoblasts; so, the cell sheet prepared with skeletal myoblasts and fibroblasts has higher VEGF production levels per cell depending on the relative proportion of skeletal myoblasts. It is believed that the VEGF produced from the cell sheet or cell sheet stack promotes the construction of a vascular endothelial network inside the cell sheet stack, and that when being transplanted onto a diseased site, the cell sheet or cell sheet stack secretes VEGF into areas around a transplantation site so that the secreted VEGF promotes angiogenesis in said areas.

The term "HGF" represents hepatocyte growth factor and generally refers to a factor characterized by being produced from fibroblasts, macrophages, vascular endothelial cells, vascular smooth muscle cells and other cells, and by acting as a paracrine factor that mainly controls the growth and functions of epithelial cells. HGF not only acts on hepatocytes but also has various actions on vascular endothelial cells, such as promotion of migration ability, morphogenesis (e.g., tubulogenesis)-inducing action, antiapoptotic action, angiogenetic action, and immune response-regulating action. In embodiments of this invention, HGF secretion is promoted through interaction between skeletal myoblasts and fibroblasts. In the case of a cell sheet or cell sheet stack each composed of skeletal myoblasts and fibroblasts, it is advantageous to incorporate skeletal myoblasts in a proportion of 90-25%, preferably 75-25%, most preferably 60-40%, since this allows preparation of a cell sheet having a high HGF production level per cell. It is believed that the HGF produced from the cell sheet or cell sheet stack promotes the construction of a vascular endothelial network and tubulogenesis inside the cell sheet stack itself, and that when being transplanted onto a diseased site, the cell sheet or cell sheet stack secretes HGF into areas around a transplantation site, promoting angiogenesis in said areas.

VEGF and HGF are both key angiogenesis-promoting factors, and two-dimensional evaluations of the culture systems for endothelial cells in a collagen gel or matrigel observed that activation of both factors affects the area occupied by a vascular structure and its total length, and has synergistic effects (refer to the following non-patent documents: Xin, et al., "Hepatocyte growth factor enhances vascular endothelial growth factor-induced angiogenesis in vitro and in vivo", Am. J. Pathol., 158, 1111-1120 (2001); and Sulpice, et al., "Cross-talk between the VEGF-A and HGF signaling pathways in endothelial cells," Biol. Cell, 101, 525-539 (2009)). Thus, a cell sheet or cell sheet stack each producing both VEGF and HGF is also acceptable. Production of both VEGF and HGF is preferred in that this leads to synergism, promoting a morphogenesis-inducing action and an angiogenetic action as exemplified by enhanced formation of a vascular endothelial cell network and tubulogenesis in a cell sheet or cell sheet stack. In the case of a cell sheet or cell sheet stack composed of skeletal myoblasts and fibroblasts, it is advantageous to incorporate skeletal myoblasts in a proportion of 75-25%, preferably 60-40%, since this allows preparation of a cell sheet having a high VEGF/HGF production level per cell. It is believed that the VEGF and HGF produced from the cell sheet or cell sheet stack promotes the formation of a high-density vascular endothelial network and tubulogenesis inside the cell sheet stack, and that when being transplanted onto a diseased site, the cell sheet or cell sheet stack secretes VEGF and HGF into areas around a transplantation site, promoting angiogenesis in said areas.

The number of cells to be seeded in the process of preparing a cell sheet by the method of the present invention varies with the animal species from which the cells to be used originate and the type of said cells. In order to prepare a cell sheet composed of either skeletal myoblasts or fibroblasts, or both of them according to an embodiment of this invention, the seeding concentration per cell sheet is advantageously 1.0×10⁵ to 7.0×10⁵ cells/cm², preferably 1.15×10⁵ to 4.6×10⁵ cells/cm², more preferably 1.5×10⁵ to 4.3×10⁵ cells/cm², and most preferably 2.0×10⁵ to 3.9×10⁶ cells/cm². In the case of preparing a cell sheet or cell sheet stack each having a vascular endothelial cell network, a cell seeding concentration of 1.0×10⁵ cells/cm² or lower is undesirable from the viewpoint of working of this invention, because such a concentration results in low cell density which causes too rapid migration of vascular endothelial cells and makes it difficult to construct a vascular endothelial cell network. A cell seeding concentration of 6.0×10⁵ cells/cm² or higher is also undesirable from the viewpoint of working of this invention, because such a concentration retards migration of vascular endothelial cells, preventing construction of an adequate vascular endothelial cell network.

It is believed that the above-mentioned mobility of a cell sheet stack may serve as an indicator of a cell sheet intended for not only the purpose of the present invention but also, for example, transplantation. As will be described in the EXAMPLES section, if the mobility of a cell sheet stack is high to some extent, blood vessels will have a correspondingly easier access from a recipient's body, making the cell sheet stack correspondingly more compatible with the body after it is transplanted. A vascular network is also expected to have an easier access in other types of cells if the stack has high mobility.

In the process of preparing the cell sheet stack, the position, order and frequency of laminating monolayer cell sheets are not particularly limited, and can be varied as appropriate depending on the tissue to be coated or replaced, typically through the use of a synovium-derived cell sheet having strong adhesion. The laminating frequency is advantageously 10 times or less, preferably 8 times or less, and more preferably 4 times or less. An extremely high laminating frequency is undesirable because this makes it difficult to deliver sufficient amounts of oxygen and nutrients to cell sheet layers in the center of the stack. This problem can be prevented by constructing a vascular network in the cell sheet stack. The method for constructing a vascular network is not particularly limited. For example, in the process of preparing a cell sheet stack, a cell sheet consisting of vascular endothelial cells or a cell sheet containing vascular endothelial cells in a predetermined proportion may be sandwiched between the cell sheets prepared by the method of this invention, placed on the topmost layer, or laid under the bottommost layer, or alternatively vascular endothelial cells may be seeded on a culture substrate beforehand and cell sheets may be stratified on the seeded vascular endothelial cells. In the case of preparing a cell sheet stack by stratifying cell sheets on the vascular endothelial cells that have been seeded on a culture substrate, the number of vascular endothelial cells to be seeded varies with the type(s) of cells to be stratified and the type of vascular endothelial cells; for example, the number of those cells is advantageously 0.1×10⁴ to 5.0×10⁴ cells/cm², preferably 0.3×10⁴ to 3.0×10⁴ cells/cm², most preferably 0.5×10⁴ to 2.0×10⁴ cells/cm². It is undesirable that the number of those cells is less than 0.1×10⁴ cells/cm² or more than 5.0×10⁴ cells/cm², because no adequate vascular endothelial cell network is formed in the former case and too dense a vascular endothelial network structure is formed in the latter case.

The method for preparing a cell sheet stack according to the present invention is also not particularly limited; for example, the cell sheet stack can be prepared by detaching cultured cells in the form of sheet and placing one sheet of the cultured cells on another using a cultured cell transfer tool dependent on the need. In the process, the temperature of a medium is not particularly limited as long as it is below the upper critical solution temperature, if any, of the above-mentioned polymer applied to the surface of a culture substrate or it is above the lower critical solution temperature, if any, of said polymer. Needless to say, it is inappropriate to perform culture in a low temperature range where no cultured cells grow or in a high temperature range where cultured cells die. The culture conditions other then temperature may be pursuant to conventional protocols and are not particularly limited. For example, the medium to be used may be a basal medium for vascular endothelial cells, a basal medium for skeletal myoblasts, a basal medium for fibroblasts, a basal medium for use in culturing a wide variety of attachment cells, or a medium to which at least one cytokine involved in angiogenesis is added later. The media tailored to cell type have formulations that depend on their respective cell culture characteristics (e.g., cytokine(s) added). In particular, the basal medium for vascular endothelial cells has an angiogenesis-related cytokine added thereto and is thus more expensive than the basal medium for use in culturing a wide variety of attachment cells. According to the present invention, the cell sheet stack itself produces an angiogenesis-promoting cytokine, so even in the presence of any basal medium having no angiogenesis-promoting cytokine added thereto, a vascular endothelial cell network is constructed in the cell sheet stack. Thus, this invention makes it possible to prepare a cell sheet stack having a vascular endothelial cell network formed therein even in the presence of a low-cost basal medium, leading eventually to reduction in the cost of transplantation. Again, since the cell sheet stack itself secretes an angiogenesis-promoting cytokine, it also promotes angiogenesis in areas around a transplantation site and can thus be expected to serve as a graft with high therapeutic efficacy. The above-mentioned media may be those which have a known serum such as fetal bovine serum (FCS) added thereto or may be serum free media having no such serum added thereto. The culture cell transfer tool to be used is not particularly limited as long as it can pick up a detached cell sheet; and examples include membranes, plates, and sponges as exemplified by porous membranes, papers, and rubbers. Alternatively, any tool that includes a handle and has a membrane, plate or sponge (e.g., porous membrane, paper or rubber) attached thereto may be used to facilitate the stratifying process.

The cell sheet stack of the present invention is characterized in that the cultured cell sheets detached from a cell culture substrate coated with a temperature-responsive polymer and picked up using a cultured cell transfer tool depending on the need are not damaged by a protease as typified by dispase or trypsin during culture, that the basement membrane-like protein formed between the cells and the substrate during culture is also not enzymatically broken down, and that the cell sheet maintains a cell-cell desmosome structure so that it has high strength with few structural defects.

In the present invention, the method for using a cell sheet stack having a vascular endothelial network constructed therein is not particularly limited; for example, with myoblasts being selected as the cells constituting the cell sheet stack, and fluorescence-labeled vascular endothelial cells being selected as the labeled cells, the migration of the labeled cells is traced with a fluorescence microscope, whereby the construction of a vascular network can be evaluated.

The cells of the present invention are not limited, and examples include those derived from animals, insects and plants, and bacterium. Among them, animal cells are advantageous because many types of those cells are commercially available. Examples of the origin of animal cells include, but are not particularly limited to, human, monkey, dog, cat, rabbit, rat, nude mouse, mouse, guinea pig, pig, sheep, Chinese hamster, cow, marmoset, and African green monkey. The above-mentioned cells may also be those which are obtained by differentiation from embryonic stem cells (ES cells) or induced pluripotent stem cells (iPS cells) but are not limited to these cells at all.

The cells to be used in the present invention are not particularly limited; for example, they may be those cells which are fluorescence-stained and/or dye-stained using at least one technique such as reagents, proteins, or genes.

If the cell sheet stack referred to in the present invention is used in a human, the transplanted cell sheet stack will then express its capability in the human body for a long time. The expression level of its capability can be controlled by either the size or shape of the detached cell sheet stack, or both of them. For example, when cardiomyocytes, cardiomyoblasts, myoblasts, or mesenchymal stem cells are selected as the cells constituting the cell sheet stack, the cell sheet stack is used for the purpose of treatment of various diseases accompanied by a cardiac disease or disorder selected from the group consisting of cardiac failure, ischemic heart disease, myocardial infarction, cardiomyopathy, myocarditis, hypertrophic cardiomyopathy, dilated phase of hypertrophic cardiomyopathy, and dilated cardiomyopathy, or reconstruction of a myocardial wall; the site for transplantation is determined as appropriate depending on the type of cell to be used, and is not particularly limited.

### EXAMPLES

The present invention will now be described in more detail with reference to Examples, but should not be limited thereto.

### [Example 1]

A co-culture system of vascular endothelial cells and a myoblast sheet was constructed (refer to the following patent document: International Patent Publication No. WO 2010/101225). FIG. 1 shows the method for quantitatively evaluating a vascular endothelial cell network formation in a cell sheet. The network of vascular endothelial cells (green) constructed in a cell sheet is subjected to image processing to determine the network length (L) and the number of tips in the network (N_{T}). The network length (L) is divided by the number of tips (N_{T}). The resulting value (L/N_{T}) is compared with the corresponding values of other cell sheets to thereby evaluate the extent of vascular endothelial network formation.

### (Analysis of the behavior of vascular endothelial cells in high-density and low-density myoblast sheets)

By using the above-described system, each type of cell sheets having different cell densities is co-cultured with endothelial cells to investigate the influences of the different cell densities on endothelial cell network formation. Further, the shape and degree of binding of endothelial cells were observed to predict network formation processes under different conditions.

### (Experimental conditions)

Monolayer myoblast sheets each having a seeding density of 1.15×10⁵ cells/cm² (very low density), 2.3×10⁵ cells/cm² (low density), or 4.6×10⁵ cells/cm² (high density) were prepared and stained with CellTracker Orange (red), and then 5 layers of each sheet were stacked. After being cultured for 96 hours, endothelial cells were stained with an anti-CD31 antibody to observe their networks (FIGs. 2, 3, 4 and 5). The experimental conditions are as described below.

### (Primary cell culture)

Cell culture was conducted using a polystyrene culture vessel (225 cm², T-flask) manufactured by Coming Inc. In the process of culturing myoblasts, laminin (Sigma) was applied to the culture surface of the vessel. A laminin-coated surface was prepared by applying a solution of laminin diluted 20-fold with a phosphate buffer solution (PBS; Sigma) at a volume of 1 mL per 25 cm² of the culture surface, performing incubation at 37°C for 1 hr in the presence of 5% CO₂ to cause proteins to adhere onto the surface, and then washing the surface with PBS.

### (Monolayer passage cultures)

In this experiment, scaffold-dependent human skeletal myoblasts (Camblex) and normal human umbilical vein endothelial cells (Lonza) were each cultured in a CO₂ incubator (MCO-17AI; Sanyo Electric Co., Ltd.) at 37°C under a 5% CO₂ atmosphere. The myoblast culture was performed using a Dulbecco's modified Eagle's medium (DMEM; Sigma) supplemented with 1 vol% antibiotic/antimycotic 100X (Invitrogen), 2 vol% 1 M HEPES buffer (Sigma), and 10 vol% fetal bovine serum (hereinafter FBS; GIBCO) (this medium is hereinafter referred to as DMEM (0% FBS)). In the process of performing the primary cell culture and passage cultures, a trypsin solution containing 0.1 % trypsin (Sigma) and 0.02% EDTA (ethylenediamine tetra-acetic acid; Sigma) was added dropwise at a volume of 1 mL per 1 cm² culture area, and reaction was allowed to proceed at 37°C for 3 min to detach the cells. To the cells in a suspended state, a trypsin inhibitor solution (Wako Pure Chemical Industries, Osaka, Japan) was added in the same amount as that of trypsin to terminate an enzymatic dispersion reaction. Centrifugation was performed at 1000 rpm at room temperature for 5 min to harvest the cells, which were then resuspended in the medium. The resulting cell suspension was seeded onto the culture surface at a viable cell concentration of 1.0×10³ cells/cm², and the medium was added at a depth of 2 mm. Medium replacement was performed every 24 hr.

The medium used in the culture of endothelial cells was EGM-2 (Lonza; hereinafter EBM). In the process of performing the primary cell culture and passage cultures, a trypsin solution containing 0.1 % trypsin (Sigma) and 0.02% EDTA (ethylenediamine tetra-acetic acid; Sigma) was added dropwise at a volume of 1 mL per 1 cm² culture area, and reaction was allowed to proceed at 37°C for 5 min to detach the cells. To the cells in a suspended state, a trypsin inhibitor solution (Wako Pure Chemical Industries, Osaka, Japan) was added in the same amount as that of trypsin to terminate an enzymatic dispersion reaction. Centrifugation was performed at 1450 rpm at room temperature for 5 min to harvest the cells, which were then resuspended in the medium. The resulting cell suspension was seeded onto the culture surface at a viable cell concentration of 2.5×10³ cells/cm², and the medium was added at a depth of 2 mm. Medium replacement was performed every 48 hr.

### (Co-culture of a myoblast sheet and endothelial cells)

The cell culture substrates used were a 35 mm polystyrene culture dish (Corning) and a temperature-responsive culture vessel (24-well multiwell; CellSeed). The temperature-responsive culture vessel has a culture surface which is a polystyrene surface graft-polymerized with N-isopropylacrylamide. Since the surface reversibly becomes hydrophobic or hydrophilic at a temperature below or above its critical point of 32°C, the cells can be easily detached from the culture surface through temperature change while they are left to adhere to each other.

The myoblast sheets were prepared as follows. Myoblasts were seeded onto each of the temperature-responsive culture vessels at a density of 1.15×10⁵ cells/cm², 2.3×10⁵ cells/cm², or 4.6×10⁵ cells/cm² such that they would be confluent after preincubation at 37°C for 24 hr. After 24 hr, the cells were incubated at 20°C for 30 min under a 5% CO₂ atmosphere to cause them to detach, and the detached cell sheets were harvested with a gelatin gel from above. These sheets each served as a monolayer cell sheet, and they were repeatedly subjected to detachment and harvesting processes, whereby multilayer cell sheets were prepared.

A sheet co-culture system was constructed by the following procedure. Endothelial cells were cultured in a 35 mm culture dish for 24 h beforehand. In the process, the medium used was EBM supplemented with 20% FBS and the density of endothelial cells seeded was 0.5×10⁴ cells/cm². After the preliminary culture, each of the multilayered myoblast sheets was placed on the dish containing the endothelial cells, and the dish was left to stand at 20°C for 2 h in a humidified atmosphere (so that the cell sheet was transferred onto the dish). Then, after DMEM supplemented with 10% FBS was introduced, the dish was left to stand at 37°C for 1 h (to dissolve the gelatin), and the medium was replaced with new one, whereupon the system was ready for subsequent culturing.

Cytoplasmic staining of the myoblasts was performed before preparation of their sheets for the purpose of knowing the height of the tissue as well as color-coding the sheets when investigating the mobility of the myoblasts. A trypsin solution containing 0.1 % trypsin (Sigma) and 0.02% EDTA (ethylenediamine tetra-acetic acid; Sigma) was added dropwise at a volume of 1 mL per 1 cm² culture area, and reaction was allowed to proceed at 37°C for 3 min to detach the cells. To the cells in a suspended state, a trypsin inhibitor solution (Wako Pure Chemical Industries, Osaka, Japan) was added in the same amount as that of trypsin to terminate an enzymatic dispersion reaction. Centrifugation was performed at 1000 rpm at room temperature for 5 min to harvest the cells, which were then suspended in a serum-free medium. To the resulting cell suspension, 10 *µ*M CellTracker Orange CMTMR (Molecular Probes) (or CellTracker Green CMFDA (Molecular Probes)) diluted with the same amount of the serum-free medium was added so as to give 5 mM CellTracker dye. After the suspension was left to stand at 37°C under a 5% CO₂ atmosphere for 15 min, centrifugation was performed at 1000 rpm at room temperature for 5 min to harvest the cells, which were then resuspended in DMEM supplemented with 10% FBS; thereafter, the suspension was seeded at a concentration of 1.15×10⁵ cells/cm², 2.3×10⁵ cells/cm², or 4.6×10⁵ cells/cm².

Antibody staining with Anti-CD31 (mouse monoclonal anti-human CD31; DAKO) was done to identify and observe the endothelial cells in the tissue where myoblasts and endothelial cells coexisted. CD31 is an antibody that recognizes a glycoprotein with a molecular weight of 100 kD which is present in endothelial cells. After the cells were washed with PBS, a 4% paraformaldehyde/phosphate buffer solution (Wako Pure Chemical Industries) was added and the cell suspension was left to stand at 4°C overnight to immobilize the cells. The culture surface was washed with PBS twice, and then 0.1% Triton X-100 diluted with PBS was added to render the cells permeable. After washing with PBS twice, the cells were immersed for 1 hr in 0.1% bovine serum albumin (Wako Pure Chemical Industries) diluted with PBS. Subsequently, the cells were immersed in an anti-CD31 antibody diluted 40-fold with 0.1 % bovine serum albumin and the cell suspension was left to stand at 4°C overnight. The cells were washed with PBS twice and immersed at room temperature for 1 hr in a secondary antibody (Alexa Fluor® 488 goat anti-mouse IgG; Molecular Probes) diluted 200-fold with 0.1% bovine serum albumin. After washing with PBS twice, one or two drops of SlowFade (Molecular Probes) were added, and a cover glass (IWAKI) was placed on top. Three-dimensional images were acquired using a confocal microscope (EX-20; Olympus) to observe the cells.

### (Procedure for evaluating the behavior of the cells)

Quantitative evaluation of vascular networks was performed by the following procedure. The network photographs taken using the confocal laser microscope were processed using Image ProPlus to perform two-dimensional image analysis of the networks (FIG. 1). The images were binarized with a visually determined threshold and thinned using a morphological filter to count the network lengths (L) and the numbers of tips (N_{T}). Based on the thus-obtained results, individual networks were compared.

After culture for 96 h, the values for endothelial networks were quantitatively evaluated based on the respective network degrees (value of network length divided by number of tips). As a result, no network was formed in the very low-density sheet, so analysis was impossible (FIG. 2). The averages of L/N_{T} were 1.12 mm/tips and 0.54 mm/tips in the low-density and high-density sheets, respectively -- the average for the high-density sheet was about half that for the low-density sheet (FIGs. 3 and 4). No difference was observed in network length L (mm), but the number of tips N_{T} (tips) for the high-density sheet was about twice that for the low-density sheet.

FIG. 5 shows the distributions of endothelial cells in the individual layers after culture for 96 h. About 70% of endothelial cells were present in the fifth layer of the low-density sheet, whereas many of endothelial cells were distributed in the first and fifth layers of the high-density sheet.

According to FIGs. 3 and 4, there was no difference in network length L (in mm) between the high-density (4.6×10⁵ cells/cm²) and low-density (2.3×10⁵ cells/cm²) sheets, so it is believed that the numbers of endothelial cells were the same between these sheets. In contrast, the number of tips N_{T} (in tips) was larger in the high-density sheet, so it is believed that the linkage between endothelial cells was poorer in the high-density sheet. Endothelial cells move in sheets together with myoblasts, forming networks in the horizontal direction. However, it is believed that in the high-density sheet, the cell mobility was lower than in the low-density sheet, thereby impairing migration of endothelial cells in the horizontal direction, which in turn led to a poorer cell linkage in the network.

Endothelial cell aggregates were observed in the fifth layers of both low-density and high-density sheets. The reason why cell aggregates were formed may be because the endothelial cells that migrated in the sheets reached the top of the sheets, where some of those cells then grew. In the high-density sheet, fewer cells were distributed in the fifth layer than in the low-density sheet, so it is believed that migration of endothelial cells in the vertical direction was impaired (FIG. 5).

### [Example 2]

### (Cytokine production ability of myoblasts depending on their cultured states)

Five-layered cell sheets are three-dimensional tissues where monolayer sheets formed at confluence are layered three-dimensionally, so it can be said that they are also in a three-dimensionally dense and confluent state. The cells in this state have temporarily lost their growth ability due to contact inhibition. In order to understand the cytokine production of sheet-forming cells three-dimensionally cultured in a confluent state, the cytokine characteristics in three-dimensional culture systems were compared with those in simpler systems, i.e., ordinary two-dimensional culture systems. It is considered that the cells form any of the following hierarchical structures -- low density, high density, sheet and layered sheet -- depending on whether they are subjected to sparse culture, dense culture, two-dimensional culture, or three-dimensional culture. Cytokine productions per cell in these hierarchical structures were investigated.

Myoblasts were seeded at densities of 1.0×10⁴ cells/cm², 8.0×10⁴ cells/cm², and 2.3×10⁵ cells/cm² (this density was the same as that of a cell sheet). At an early stage, 48 h, of culture (i.e., 48 h after the start of culture), culture media were collected to investigate VEGF and HGF production levels. These levels were standardized using the numbers of cells calculated by trypan blue staining, so that the average production levels per cell were compared.

FIG. 6 shows the VEGF and HGF production levels per cell which were determined using the media collected 48 h after culture. These levels were calculated by determining the total production levels from the concentrations detected by ELISA and the volumes of the media and standardizing the total production levels with the total number of cells determined from the above-mentioned cell densities and the area of the culture dishes. For the purpose of comparison, this figure also shows the values obtained by standardizing the production levels of the 5-layered myoblast sheet at the time of 48 h of culture using the number of cells. The VEGF production level per cell was higher as the cell density was higher; in particular, this level was the highest at a confluent cell density (1.9×10⁵ cells/cm²). The results show that myoblasts in a dense, confluent state have an enhanced VEGF production ability. The value for the culture of the 5-layered sheet was also similar to that of the confluent cells; so it is believed that the sheets prepared exclusively with confluent cells are effective as a graft material in terms of VEGF secretion ability (secretion efficiency).

In contrast, as shown in FIG. 7, it was found that like the above-mentioned results, the production levels of both VEGF and HGF were increased with the increase in the number of cell sheets layered though the cell sheets were prepared in different ways. The reason for these results may be because there occurs a difference in the migration ability of the cells due to change in cell density in the cell sheets, whereupon a change in metabolism occurs to cause variations in production level.

The above-mentioned results suggested that in the process of transplantation of myoblasts, it is effective in terms of cytokine production level in a diseased site to provide the cells in the form of cell sheet, in particular layered cell sheet, rather than single cell.

It has been found out that a group of myoblasts collected and isolated from a skeletal muscle tissue also include fibroblasts (Rhoads, et al., "Satellite cell-mediated angiogenesis in vitro coincides with hypoxia-inducible factor pathway", Am J. Physiol. Cell Physiol., 296, C1321-C1328, (2009)). It is known that fibroblasts occur in a variety of tissues and migrate to a diseased site upon an inflammatory reaction, contributing to healing. In particular, it is believed that fibroblasts actively produce extracellular matrices (ECM) and cytokines, thereby contributing to maintaining the structures of biological tissues and the physiological states of cells (Tomasak, et al., "Myofibroblasts and mechano-regulation of connective tissue remodeling", Nat. Rev. Mol. Cell Biol., 3, 349-363 (2002)). Accordingly, there is a possibility that the fibroblasts present in a cell sheet may affect the mechanical properties of the entire cell sheet as well as the migration and differentiation of myoblasts in the cell sheet. However, no relationship has been identified between the properties of the entire sheet and the proportion of fibroblasts present therein. There is a possibility that the expression/formation patterns of membrane proteins and ECM which are involved in intercellular adhesion and the secretion pattern of cytokines contributing to cell migration ability may be changed in the presence of a high proportion of fibroblasts.

Thus, 5-layered cell sheets prepared from a mixture of myoblasts and dermal fibroblasts were seeded on polystyrene surfaces and subjected to sheet culture, and culture media were collected every 24 hours. Cytokine secretion levels were determined from the collected culture media by ELISA (enzyme-linked immunosorbent assay). The proportion of myoblasts was varied in the order of 100%, 75%, and 50% to investigate the dependence of myoblasts on purity.

Protein-level evaluation was made of cytokines that are said to have one of the paracrine effects of the cell sheets, i.e., angiogenesis-promoting effect. Specifically, VEGF and HGF were chosen from growth factors as the targets that, after sheet transplantation, provides a cardiac disease site with a stimulated migration ability of vascular endothelial cells (and endothelial progenitor cells present in the blood), thereby promoting angiogenesis in the transplanted tissue. Culture media were collected every 24 hours, the concentrations of detected factors were determined from the absorbances obtained from the media collected at predetermined time points, and the concentrations were multiplied by the volumes of the media to thereby obtain the masses of respective factors produced by sheet samples for 24 hours; these masses were evaluated (FIGs. 8a and 8b).

### - Experimental conditions

Cells: Human skeletal myoblasts, Np 6 (Lot No. 4F1618; Lonza, Inc.)
   Human dermal fibroblasts, Np 8 (Lot No. 3C0243; Cascade Biologics, Inc.) Medium: DMEM (10% FBS)
Culture surfaces: Polystyrene culture dish (for culture of 5-layered sheets); 24-well temperature-responsive culture surface (for sheet formation; CellSeed, Inc.); laminin-coated polystyrene culture surface (for growth of myoblasts)
Culture environment: 37°C, 5% CO₂ in air
Seeding density: 2.3×10⁵ cells/cm² (for sheet formation)
Sheet culture periods: 48 h, 168 h
Medium volume: 1.76 mL (for culture of 5-layered sheets; medium depth 0.2 cm)
Medium replacement: Every 24 hours
Samples collected: Culture medium collected every 24 hours (ELISA)
Targets: VEGF (vascular endothelial growth factor)
   HGF (hepatocyte growth factor)

As shown in FIGs. 8a and 8b, the concentrations of VEGF and HGF detected 48 hours after culture were in the range of 500 to 5000 pg/mL -- these orders of magnitude partially agreed with those in which the stimulatory effects of the addition of VEGF and HGF on, for example, the migration and network formation of endothelial cells can be observed (VEGF: 3000 pg/mL-10 ng/mL; HGF: 2500-5000 pg/mL) (Pepper, et al., "Potent synergism between vascular endothelial growth factor and basic fibroblast growth factor in the induction of angiogenesis in vitro", Biochem. Biophys. Res. Com., 189, 824-831 (1992); and Bussolino, et al., "Hepatocyte growth factor is a potent angiogenic factor which stimulates endothelial cell motility and growth", J. Cell Biol., 119, 629-641 (1992)). As the proportion of incorporated fibroblasts increased, VEGF production decreased but HGF production increased. These significant changes were almost linear with respect to the proportion of incorporated fibroblasts; so this observation suggested that VEGF and HGF are mainly secreted by myoblasts and fibroblasts, respectively. It is believed that incorporation of fibroblasts into a myoblast sheet is undesirable for endothelial cells in terms of their VEGF protein secretion ability but desirable for them in terms of their HGF secretion ability; so there may be an optimum balance of mixing myoblasts and fibroblasts in a cell sheet.

As mentioned above, there was observed a modulation of different cytokine characteristics due to incorporation of fibroblasts in the process of culture of the 5-layered myoblast sheets. In this connection, a group of myoblasts inherently include fibroblasts, and the population of myoblasts was evaluated to be 78.2±2.1% by double antibody staining (myoblasts: desmin; fibroblast: TE-7 antigen). Thus, the cytokine production levels at an early stage, i.e., 48 h, of culture as shown in FIGs. 8a and 8b were replotted in FIGs. 8c and 8d with respect to the actual population of myoblasts R_{M}.

The effect of the presence of dermal fibroblasts on myoblasts in a high-density culture which is a mimetic system for the 5-layered cell sheets was investigated. Cell groups were prepared by mixing myoblasts and fibroblasts at ratios of 100:0, 75:25, 50:50, 25:75, and 0:100 (in the system for the cell sheets, it was impossible to form a cell sheet with a group of cells containing dermal fibroblasts in a proportion higher than 50%). These groups of cells were seeded at a high density (1.9×10⁵ cells/cm²), and the media at 24-48 h of culture (i.e., 24-48 hours after the start of culture) were collected to determine VEGF and HGF production levels. The levels were standardized by the cell count to enable comparison of the production levels per cell. The results are shown in FIG. 10. The VEGF and HGF productions in the 5-layered sheets at 48 h of culture, which are shown for comparison, showed a similar dependence on cell mixing ratio to the productions upon high-density culture. The VEGF production level showed a monotonic decrease with increase in the population of fibroblasts, but the HGF production level showed the highest value in the presence of 50% fibroblasts. Accordingly, it is suggested that myoblasts may mainly contribute to VEGF production but HGF may be produced cooperatively through interaction between myoblasts and fibroblasts (FIG. 9).

### [Example 3]

### (Endothelial cell network formation in myoblast-fibroblast mixture sheets)

Myoblast sheets or myoblast-fibroblast mixture sheets were each placed on endothelial cells to perform co-culture. It was found that the characteristics of a cell sheet itself, such as mobility in sheet and cytokine secretion pattern, are modulated by incorporation of fibroblasts; so this may provide vascular endothelial cells with modulation of the surrounding environment. Therefore, the possible influence of mixed cell sheets on network structure formation was investigated (FIG. 10).

FIG. 11 shows the results of the network formation using skeletal myoblast-dermal fibroblast mixture sheets. At the time of 48h of co-culture (i.e., 48 hours after the start of co-culture of the skeletal myoblast-dermal fibroblast mixture sheets and vascular endothelial cells), the 50% myoblast-mixed sheet formed a denser, more branched, and more homogenous network structure than the 100% myoblast sheet.

In this study, fibroblasts derived from a human skeletal muscle tissue were difficult to obtain, so the effect of the presence of fibroblasts on myoblast sheets has been investigated by incorporating fibroblasts derived from a human dermis. It has been reported that analyses by cell morphology, cytoskeleton distribution and specific markers proved that fibroblasts derived from the skeletal muscle, dermis, lung, and subcutaneous tissue all have similar morphological characteristics (Xin, et al., "Hepatocyte growth factor enhances vascular endothelial growth factor-induced angiogenesis in vitro and in vivo", Am. J. Pathol., 158, 1111-1120 (2001)). However, myoblasts contain fibroblasts derived from a skeletal tissue, and the actual extent of the presence of fibroblasts varies among lots. There is a possibility that the proportion of fibroblasts present may increase in the process of cell growth in a recipient's body which is indispensable in regenerative medicine. Therefore, in order to evaluate the characteristics of cell sheets serving as a graft material, it is important to obtain a finding on the influence exerted by inherent fibroblasts derived from a skeletal muscle. Thus, the influence of fibroblasts on network formation was investigated using the cells stored by the medical school project research team.

The myoblast lots collected from the same patient for the purpose of the clinical study of sheet transplantation (two lots of myoblasts at purities of 80% (Lot A) and 10% (Lot B) at the time of sheet formation) were mixed at ratios of 100:0, 75:25, and 50:50 to prepare cell suspensions each containing myoblasts in proportions of 80%, 62%, and 45% (antibody staining confirmed that aside from the myoblasts, only the skeletal muscle-derived fibroblasts were contained in the cell suspensions). Five-layered cell sheets were prepared using these cell suspensions and were co-cultured with endothelial cells for 48 hours to investigate network formation at this time point (FIG. 12).

### - Experimental conditions

Cells: Human skeletal myoblasts, Np 3 (Lot hMB21A; desmin-positive 81%)
   Human skeletal myoblasts, Np 3 (Lot hMB21; desmin-positive 0%)
   Human umbilical vein endothelial cells, Np 4 (Lot 4F0709; Lonza, Walkersville, MD, USA)
Medium: DMEM (10% FBS; myoblasts)
Culture surfaces: Polystyrene culture dish (for culture of 5-layered sheets); 24-well temperature-responsive culture surface (for sheet formation; CellSeed, Inc.); laminin-coated polystyrene culture surface (for growth of myoblasts)
Culture environment: 37°C, 5% CO₂ in air
Seeding density: 2.3×10⁵ cells/cm² (for sheet formation)
Medium volume: 1.76 mL (for culture of 5-layered sheets; medium depth 0.2 cm; DMEM/10% FBS)
Medium replacement: Every 24 hours
Co-culture period: 48 hours
Staining reagents: Anti-CD-31 antibody (endothelial cell marker), CellTracker Orange CMTMR (for staining of sheet-forming cells and cytoplasmic staining)
Observation: Confocal laser microscope (X10)

FIGs. 12 and 13 show the observational photographs taken using a confocal laser microscope. It appeared that as compared with the co-culture with the 81% myoblast sheet, the co-cultures with the 61% and 41 % myoblast sheets having a higher fibroblast content yielded formation of denser and more homogeneous network structures. It was found to be preferable to form a cell sheet using a mixture of myoblasts and fibroblast in a myoblast proportion ranging from 100% (inclusive) to 40% (inclusive). It is believed that as far as at least the network formation in myoblast sheets is concerned, the skeletal muscle-derived fibroblasts exhibit a similar effect to the dermis-derived fibroblasts.

It has been confirmed through the determination of cytokine production in cell sheets that the VEGF and HGF production patterns are reversely modulated each other in association with incorporation of fibroblasts into a group of myoblasts. These cytokines are both said to be key angiogenesis-promoting cytokines; for example, two-dimensional evaluations of the culture systems for endothelial cells in collagen gel and matrigel observed the synergistic effects of activation of both factors on the area occupied by a vascular structure and its total length. Further, it is believed that since HGF is involved in cytoskeleton-related signaling to endothelial cells, it acts on the branched structure of the network (Sulpice, et al., "Cross-talk between the VEGF-A and HGF signaling pathways in endothelial cells", Biol. Cell, 101, 525-539 (2009)). Accordingly, it is suggested that these factors both may play a complementary role in signaling for angiogenesis in endothelial cells and may be involved in the effect on network formation which was observed upon co-culture with the mixed sheets (FIG. 14).

It was revealed that optimization of the relative proportions of myoblasts and fibroblasts as well as cell seeding concentrations results in not only increased cytokine productions inside and outside a cell sheet stack but also enhanced construction of a vascular endothelial cell network. It is known that when a cell sheet having a vascular endothelial cell network constructed therein is transplanted, the cell sheet quickly establishes a linkage with vascular networks around a transplantation site, exhibiting high therapeutic efficacy. It is also believed that currently available therapies in patients with cardiac diseases using myoblast sheets are based on the paracrine effects typically produced by cytokines secreted from the sheets. Therefore, it is considered that the cell sheet prepared according to the present invention, which has a high cytokine production level and has a vascular endothelial cell network highly constructed therein can provide better therapeutic efficacy.

### INDUSTRIAL APPLICABILITY

The preparation method referred to in the present invention can produce a cell sheet stack that secretes an angiogenesis-promoting cytokine and/or has a vascular endothelial cell network constructed therein. This method leads to not only provision of a graft having high therapeutic efficacy but also preparation of a cell sheet stack having a higher thickness, and thus is useful for regenerative medicine for various tissues.

### REFERENCE SIGNS LIST

- R_{G}:: Proportion of green voxels in one image slice (R_{G} = ratio of green vexels)
- d:: Thickness of an imaged cell sheet stack
- R_{M}:: Proportion of myoblasts in the total number of cells
- z:: Sheet thickness [µm]
- L:: Total vascular network length in one image [mm/mm²]
- N_{T}:: Number of tips per 1 mm² of vascular network [tip/mm²]
- L/N_{T}:: The value of L divided by N_{T} [mm/tip]

## Claims

1. A cell sheet or cell sheet stack, wherein the cell sheet has an ability to produce a cytokine involved in angiogenesis promotion, and wherein cells constituting the cell sheet include at least fibroblasts as well as include skeletal myoblasts in a proportion of 10% or higher.

2. The cell sheet or cell sheet stack according to Claim 1, wherein the fibroblasts are derived from a skeletal tissue.

3. The cell sheet or cell sheet stack according to any one of Claims 1 and 2, wherein the skeletal myoblasts are contained in a proportion of 75-25%, and wherein the cytokine is a vascular endothelial growth factor (VEGF) and/or a hepatocyte growth factor (HGF).

4. A cell sheet or cell sheet stack, wherein the cell sheet or cell sheet stack according to any one of Claims 1-3 is placed on seeded vascular endothelial cells to thereby construct a vascular endothelial cell network.

5. The cell sheet or cell sheet stack according to any one of Claims 1-4, wherein said cells are collected from a biological tissue.

6. The cell sheet or cell sheet stack according to any one of Claims 1-5, wherein at least one type of cells selected from skeletal myoblasts, fibroblast, and vascular endothelial cells are derived from an autologous tissue.

7. The cell sheet or cell sheet stack according to any one of Claims 1-6, wherein said cells are fluorescence-labeled cells.

8. A method for preparing the cell sheet according to any one of Claims 1-7, wherein said cells are placed on a cell culture support having a surface coated with a polymer which changes a hydration force in a temperature range of 0-80°C, said cells are cultured in a temperature range where the polymer exhibits a weak hydration force, and then the temperature of a medium is changed to a temperature at which the polymer exhibits a strong hydration force, so that the cultured cells are detached in the form of sheet.

9. A method for preparing the cell sheet according to any one of Claims 1-7, wherein said cells are seeded at a concentration of 1.0×10⁵ to 7.0×10⁵ cells/cm² per one layer of cell sheet.

10. A method for preparing the cell sheet stack according to any one of Claims 1-7, wherein the detached cell sheet is further placed on another sheet of culture cells, or this step is repeated, so that a cell sheet stack is formed.

11. The method for preparing the cell sheet or cell sheet stack according to any of Claims 8-10, wherein the detachment from the cell culture support is achieved without protease treatment.

12. The method for preparing the cell sheet or cell sheet stack according to any of Claims 8-11, wherein upon completion of the culture, a carrier is adhered to the cultured cells and the cells are detached together with the carrier.

13. The method for preparing the cell sheet or cell sheet stack according to any of Claims 8-12, wherein the polymer which changes a hydration force in a temperature range of 0-80°C is poly(N-isopropylacrylamide).

14. The cell sheet or cell sheet stack according to any one of Claims 1-7, wherein the obtained cell sheet or cell sheet stack is used for transplantation onto the heart in a recipient's body.

15. The cell sheet or cell sheet stack according to Claim 14, wherein the obtained cell sheet or cell sheet stack thereof is used to perform transplantation intended for treatment of at least one cardiac disease or disorder selected from the group consisting of cardiac failure, ischemic heart disease, myocardial infarction, cardiomyopathy, myocarditis, hypertrophic cardiomyopathy, dilated phase of hypertrophic cardiomyopathy, and dilated cardiomyopathy, or for reconstruction of a myocardial wall.
